Europäisches Patentamt

European Patent Office

Office européen des brevets

(11) Publication number: **0 075 415**
**A2**

# EUROPEAN PATENT APPLICATION

(21) Application number: **82304735.2**

(22) Date of filing: **09.09.82**

(51) Int. Cl.³: **A 61 B 1/06,** G 02 B 5/16

(30) Priority: **10.09.81 JP 143354/81**

(43) Date of publication of application: **30.03.83**
**Bulletin 83/13**

(84) Designated Contracting States: **AT BE CH DE FR GB IT
LI NL SE**

(71) Applicant: **OLYMPUS OPTICAL CO., LTD.,**
**43-2, 2-chome, Hatagaya Shibuya-ku, Tokyo 151 (JP)**

(72) Inventor: **Shishido, Yoshio, 2-1-30-512 Fuchinobe
Sagamihara-shi, Kanagawa-ken (JP)**

(74) Representative: **Kennedy, Victor Kenneth et al, G.F.
REDFERN & CO. Marlborough Lodge 14 Farncombe
Road, Worthing West Sussex BN11 2BT (GB)**

(54) **Endoscopes provided with perspective illumination attachments.**

(57) A perspective viewing endoscope (11) having a perspective illumination attachment fitted to produce shades corresponding to concavo-convex surface details of an object within a body cavity, so that the object having the concavo-convex surface details can be seen to be three-dimensional features defined by shading.

In an endoscope wherein illuminating light is projected into a body cavity to observe or photograph the interior of the body cavity by the reflected light if the interior of the body cavity is illuminated from only one direction by substantially parallel illuminating light beams projected from a light-projecting surface, the reflected light received at an observation window positioned near the illuminating light-projecting surface prevents any shading corresponding to concavo-convex surface features of the object being formed by the illuminating light beams, and as shading cannot be observed, the image appears very flat.

In the present invention, illuminating light is projected directly from a surface (17) of an insertion sheath (13) of the endoscope (11) and also reflected by an inclined surface (24) to provide illuminating light beams dispersed over a predetermined angle and not arranged in parallel formation, whereby shading of the image corresponding to concavo-convex surface details of an object within a body cavity can be produced, and the object within the body cavity can be studied for three-dimensional features by examination of the shading.

-1-

# ENDOSCOPES PROVIDED WITH PERSPECTIVE ILLUMINATION ATTACHMENTS

This invention relates to an endoscope provided with a perspective illumination attachment.

In recent developments for diagnosis or therapy in the medical field, the use of an endoscope to observe or photograph an affected part within a body cavity has become increasingly common.

For such observation or photography the endoscope normally comprises an insertion sheath housing illuminating means to project light from a light guide to be selectively absorbed and reflected by an object, and an optical image forming means focusing the reflected light, and an observing optical system transmitting the image so that it may be utilised for observation, and photographed. As the light-beam components projected into the observing optical system formed of an objective and others are limited to lights projected from one direction, in order to clearly focus the optical image, if the illuminating light paths are substantially parallel, the optical image that is formed of an object having concavo-convex surfaces will be limited by the parallel light-paths reflected substantially parallel in the reverse direction, so that the illuminated object will appear as a flatly lit image.

In such cases, if there are concavo-convex surfaces, the light reflected by the object and projected into the observing optical system will vary in amount in dependence upon the reflecting surfaces of the concavo-convex structure, but will be hard to distinguish as an optical image when examining a relayed image or photograph with the naked eye. Therefore, illuminating devices have been used wherein illuminating light-paths are not mutually parallel but are made to be able to form shades and enlarge the contrast in response to concavo-convex surfaces, so that the concavo-convex details of the object can be known, as are disclosed, for example, in the gazette of Japanese Utility model laid-open No. 61202/1981; or the West German Utility model Registration No. 7907876; and the U.S. Patent No. 3,799,150. In the device mentioned in the gazette of Japanese Utility model laid-open No. 61202/1981, there are problems presented because the tip surface of a light guide out of which illuminating lights are projected protrudes out of an observing window and, as a result, this observing window into which reflected light is taken is relatively recessed, so that in use, unnecessary substances in the body cavity will be likely to accumulate there and so obstruct observation.

Further, in the device described in the above mentioned West German Utility model Registration No. 7907876, there are problems that, though a reflecting ring is provided, when this ring contacts a living body structure, body fluid or the like will accumulate in the ring, and the illuminating light reflecting function will be reduced and the illuminated range will become narrow.

In the device mentioned in the U.S. Patent No. 3,799,150, there are problems that, as the diameter of the tip is larger than the diameter towards the external end of the inserted part, the inserted part will not be able to be removably inserted into a trocar, which is a thrust guide tube for removably inserting the endoscope into a body cavity, and as a prism and optical fibres are used, the durability will be low.

One object of the present invention is to provide an endoscope with a perspective illumination attachment by which it is possible to provide a projecting angle of a wide range to an object, to enable shaded images corresponding to concavo-convex surface details of the object to be produced, the concavo-convex details of the object being recognisable as three-dimensional details, and the tip part of the attachment being in contact with the object being observed or photographed so that a stable optical image can be provided and any vibration can be prevented, particularly when photographing, and further, as the attachment is formed as a removable extension sheath fitted to the insertion sheath of the endoscope, there are advantages that any reflecting surface and areas be easily washed or disinfected and so kept clean, and that the optical performance does not reduce for a long time when used as an attachment on a general endoscope.

According to one aspect, the invention provides a perspective viewing endoscope provided with a perspective illumination attachment in the form of an extension sheath fitting in removable manner on the insertion sheath of an endoscope, said insertion sheath housing an illuminating optical system and an observing optical system comprising an objective system, image transmitting means and eyepiece system, so that illuminating light beams may be fed into the body cavity by the above-mentioned illuminating optical system and an object within the body cavity illuminated by the above mentioned illuminating optical system may be focused to form an image by the above mentioned objective system and transmitted to the eyepiece by the above mentioned image transmitting means to observe the body cavity interior, wherein an inner wall of the extension sheath is inclined to an illuminating light projecting surface formed at the tip of the insertion sheath within the tip of the extension sheath to provide a reflecting surface diverting into the body cavity the illuminating light beams from the inclined light projecting surface.

Aspects of the invention will now be described with reference to the drawings, in which:-

Figure 1 is an explanatory view showing details of one known conventional illuminating device;

Figure 2 is a partly sectioned schematic side view showing an extension sheath as proposed by the present invention, fitted to a perspective-viewing hard endoscope;

Figure 3 is a magnified sectional view of the tip of the embodiment shown in Figure 2; and

Figure 4 is an explanatory view showing that shades are made on an observed optical image in response to concavo-convex surface details of an object by reflected illuminating light beams.

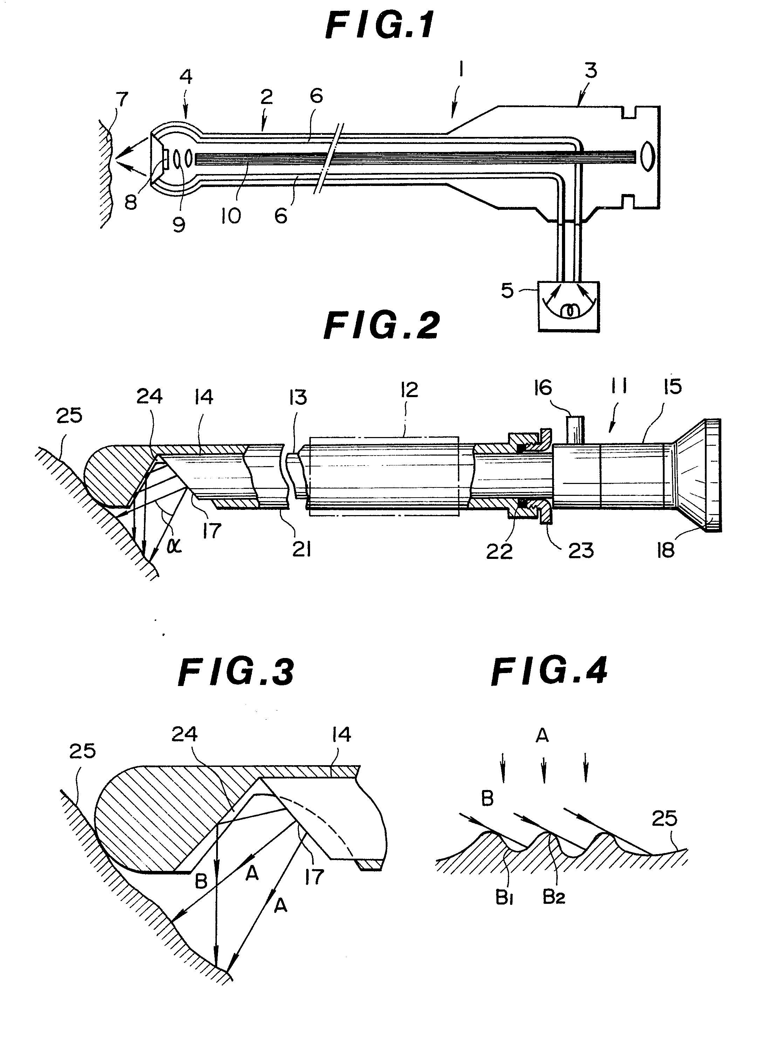

Prior to explaining details of the exemplary embodiments of the present invention, a conventional example of a known endoscope will be described with reference to Figure 1, which illustrates an endoscope 1 having an insertion sheath 2 to be inserted into a body cavity, and an operating hand grip 3 on the outer end, linked by an illuminating optical system and observing optical system inserted internally through the operating grip 3 to the tip 4 of the insertion sheath 2.

Illuminating light from an external light source 5 is fed via light guides 6 in the operating grip 3 through a universal joint or flexible cable portion. The light guides 6 transmit the light from the light source 5 to the tip 4. The illuminating optical system is so formed that the illuminating light-paths are projected toward an object 7 through the end faces of the light guides 6 at the tip of the insertion sheath.

The end faces of the light guides 6 are formed to lie on a curved surface around the spherical outer periphery of the observing optical system, in which an objective system 9 and image guide 10 are centrally arranged, the inner part of the tip opening having an observation window 8 for receiving reflected light to form an image and transmit this to the eyepiece.

In this conventional known construction, as the end faces of the light guides 6, from which the illuminating light is projected, protrudes forward of the observation window 8, which is recessed, problems arise in use because unwanted substances within the body tend to accumulate and so obstruct observation.

Further, there are problems because the diameter of the tip 4 is restricted by the size of a trocar or the like, and it will be difficult to select a projecting angle at the tip so as to intersect the axis of the optical system at a large angle, and with this restriction concavo-convex surface details of the object will not be shown clearly enough.

The present invention is designed to avoid the problems of the above mentioned conventional known example.

The perspective viewing endoscope with an additional illuminating sheath of a preferred embodiment of an endoscope constructed in accordance with the present invention will now be described with reference to Figures 2 to 4.

In the embodiment shown in Figure 2, a perspective viewing hard endoscope 11 has an illuminating optical system and observing optical system arranged with an insertion sheath 14 to be inserted into a body cavity, as guided in a trocar 12 thrust into the body wall, from an operating grip 15 at the rear end. A connector 16 to receive a universal joint coupling or flexible cord transmitting illuminating light from a light source (not shown) is formed on the operating grip 15. The light guides inserted through this connector 16 extend to the tip of the sheath 14, and the end faces of this light guide, or a glass window arranged to cover the end faces provides an inclined illuminating light-projecting surface 17, i.e. it is inclined to the optical axis to permit perspective type viewing, as in the illustration. A central observation window of glass is provided to receive reflected light, and lies in the plane of this light-projecting surface 17, and an objective system is provided internally. An optical image formed by the objective system within the end of the insertion sheath 14 is transmitted via a central portion 13 of the sheath to an eyepiece 18 provided in the operating grip 15, through an image guide and relay lens, so that the object in front of the sheath tip 14 can be observed by viewing the optical image in the eyepiece 18 directly with the naked eye, or after photographing it, using a camera fitted to the eyepiece.

The insertion sheath 13 of the hard endoscope 11 is inserted to fit in a removable perspective illumination attachment sheath (which will be referred to as an outer sheath hereinafter). This outer sheath has a cylindrical tube 21 so formed at its rear end as to be able to be sealed and engaged by a retaining screw-ring 23 provided at the rear end of the inserted sheath 13 near the operating grip 11, being sealed by an airtight O-ring 22. The above mentioned O-ring 22 prevents abdominal gases or the like from leaking, when the insertion sheath 13 of the hard endoscope 11 fitted with the outer sheath is inserted via the trocar 12 into a body cavity.

The remote end of the outer sheath is hemispherical at the tip, and has a reflecting surface 24 diagonally opposed to the inclined light-projecting surface 17 when the insertion sheath 13 of the hard endoscope 11 is inserted to bring its end 14 adjacent the surface 24, and fixed facing the aperture in the outer sheath tube 21. The field of view from the observation window is defined by marginal beam paths defining an angle $\alpha$ at the inclined surface 17, and illuminating light beams A cover this region by direct radiation, and also provide a perspective illumination due to light beam components B that are projected from the surface 17 to strike the reflecting surface 24 and pass out of the aperture almost normal to the optical axis.

The surface 24 may be formed by an inner wall surface of the outer sheath, and may be treated to improve its reflectivity by plating or coating with a material that is not attacked by body fluids or the like.

The surface 24 is not necessarily flat, but can be a curved or dish-shaped surface shaped to not disperse the light over too wide a region, and may be so formed as to reflect light on paths inclined to the axis by a predetermined angle (large enough for perspective illumination). Furthermore, the reflective surface 24 can be deliberately roughened so as to scatter reflected light in a regular manner, and give an even illuminated image, so that illumination level fluctuations on the illuminated object 25 do not depend on the state of the projecting surface 17, or the wave guide end faces, etc. A rough surface 24 can be formed by rough grinding, by forming many grooves, or by bonding many fine, highly reflective grains on the surface 24. The outer sheath end tip is made hemispherical, so that if it contacts any surface of the object that is to be observed it will not hurt the surface of an internal organ or the like within the body cavity.

In using the thus formed outer sheath fitted to the hard endoscope 11, the outer sheath is fitted to the hard endoscope 11, and is fixed at the outer end by the retaining screw-ring 23. The hard endoscope 11, thus fitted with the outer sheath, is inserted into a trocar 12 thrust into a body wall, illuminating light transmitted from a light source (not illustrated) to be projected from the surface 17 at the tip of the insertion sheath 14, and the hemispherical outer sheath is contacted with a desired surface of the object 25 by observing the state within the body cavity via the eyepiece 18.

In this state, some of the illuminating light beams projected from the surface 17 directly illuminate the surface of the object 25 (as shown by the arrows A in Figures 3 and 4) and, at the same time, other beam components B are reflected by the surface 24 and illuminate the object at a different angle.

It will now be described with reference to Figure 4 how the concavo-convex surface state of the object observed with the observing optical system is clearly shown by the additional reflected illumination.

As shown in Figure 4, with only the direct illuminating light beams A from the projecting surface 17 an optical image to be observed or photographed will be formed of the light level reflected in the reverse direction purely in response to the state of the surface of the object 25. If the object 25 is observed via the observing optical system, the optical images of the concave and convex portions of the surface, $B_1$ and $B_2$ respectively will only differ in the light levels reflected from these parts and projected into the observing optical system due to the local surface state, and therefore, even if there are concavo-convex contours, they will not be clearly discernable in the optical images.

However, by providing a perspective illumination by reflected light beams B the above-mentioned convex portion $B_2$ will be additionally illuminated, but the concave portion $B_1$ will be in shade, and thus a large contrast in light levels will be made by the concavo-convex surfaces. Therefore, the observed optical image of the object 25 illuminated by the reflected light beams in addition to the direct beams A exhibits a large contrast difference in response to the concavo-convex surface details of the object. In these drawings, the reflected illuminating light beams are in one direction, but the use of beams incident in many directions is the same.

Thus, the concavo-convex surface details of the object can be clearly known.

As the outer sheath tip can contact the surface of the object 25, the observing optical system can be kept at a fixed distance from the surface of the object 25, and any vibration can be prevented, which is of great advantage, particularly in the case of photographing. In this case, the tip of the outer sheath may be made movable forward of the illustrated position, so as to be convenient to observe or photograph the object 25. Further, this outer sheath tip is not essentially hemispherical, but may be rounded to a lesser degree. In the above description, an outer sheath provided on a perspective hard endoscope is shown. However, if the angle of inclination of the surface 24 is adjusted, the outer sheath could be applied with a straight-sight type of endoscope, as well as to the perspective type. Also, if the tube part 21 of the outer sheath is made of a flexible soft tube, the outer sheath can be applied in similar manner to a soft endoscope, the perspective view still being discernable because of the provision of separate illuminating beam components incident on common points of an object from widely divergent directions.

CLAIMS:-

1.      A perspective viewing endoscope provided with a perspective illumination attachment in the form of an extension sheath fitting in removable manner on the insertion sheath of an endoscope, said insertion sheath housing an illuminating optical system and an observing optical system comprising an objective system, image transmitting means and eyepiece system, so that illuminating light beams may be fed into the body cavity by the above mentioned illuminating optical system and an object within the body cavity illuminated by the above mentioned illuminating optical system may be focused to form an image by the above mentioned objective system and transmitted to the eyepiece by the above mentioned image transmitting means to observe the body cavity interior, wherein an inner wall of the extension sheath is inclined to an illuminating light projecting surface formed at the tip of the insertion sheath within the tip of the extension sheath to provide a reflecting surface diverting into the body cavity the illuminating light beams from the inclined light projecting surface.

2.      A perspective viewing endoscope as claimed in Claim 1, characterised in that the outer tip of the extension sheath is rounded.

3.      A perspective viewing endoscope as claimed in Claim 1, characterised in that the reflecting surface is curved, dished or provided with a rough surface so as to provide even illumination over the field of view.

4.      A perspective viewing endoscope as claimed in Claim 1, characterised in that sealing means provide a seal between the endoscope insertion sheath and the outer sheath at the rear end.

5.      A perspective viewing endoscope as claimed in
Claim 1, characterised in that the reflecting surface
is formed by an inner wall surface of the sheath,
or by a material plated or coated thereon.

6.      A perspective viewing endoscope, as claimed in
Claim 3, characterised in that the rough surface is
formed by the provision of grooves, or by bonding
fine grains on to the surface.

# FIG.1

# FIG.2

# FIG.3

# FIG.4